(19) Europäisches Patentamt
European Patent Office
Office européen des brevets

(11) EP 0 895 470 B1

(12) EUROPÄISCHE PATENTSCHRIFT

(45) Veröffentlichungstag und Bekanntmachung des Hinweises auf die Patenterteilung:
**20.09.2006 Patentblatt 2006/38**

(21) Anmeldenummer: **97954365.9**

(22) Anmeldetag: **09.12.1997**

(51) Int Cl.:
***A61K 8/44*** *(2006.01)* ***A61Q 5/10*** *(2006.01)*

(86) Internationale Anmeldenummer:
**PCT/EP1997/006860**

(87) Internationale Veröffentlichungsnummer:
**WO 1998/030197 (16.07.1998 Gazette 1998/28)**

(54) **MITTEL UND VERFAHREN ZUR DAUERHAFTEN HAARVERFORMUNG AUF BASIS VON N-VERZWEIGTKETTIG ALKYLSUBSTITUIERTEN MERCAPTOACETAMIDEN**

AGENT AND METHOD FOR PERMANENTLY SHAPING THE HAIR, BASED ON N-BRANCHED-CHAIN ALKYL-SUBSTITUTED MERCAPTO ACETAMIDES

PROCEDE ET AGENT POUR PERMANENTES A BASE DE MERCAPTOACETAMIDES SUBSTITUES PAR ALKYLE A CHAINE N-RAMIFIEE

(84) Benannte Vertragsstaaten:
**DE ES FR GB IT**

(30) Priorität: **11.01.1997 DE 19700725**

(43) Veröffentlichungstag der Anmeldung:
**10.02.1999 Patentblatt 1999/06**

(60) Teilanmeldung:
**05026660.0 / 1 639 992**

(73) Patentinhaber: **Wella Aktiengesellschaft**
**64274 Darmstadt (DE)**

(72) Erfinder:
- **LANG, Günther**
  **D-64354 Reinheim (DE)**
- **DANNECKER, Beate**
  **D-64283 Darmstadt (DE)**
- **HANEFELD, Wolfgang**
  **D-35037 Marburg (DE)**
- **WALTHER, Heiko**
  **D-35037 Marburg (DE)**

(56) Entgegenhaltungen:

| | |
|---|---|
| **EP-A- 0 514 282** | **WO-A-91/10421** |
| **DE-A- 19 618 445** | **US-A- 4 220 602** |

EP 0 895 470 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft ein Mittel zur dauerhaften Haarverformung, welches als keratinreduzierenden Wirkstoff bestimmte neue Mercaptoacetamide enthält sowie ein Verfahren zur dauerhaften Haarverformung unter Verwendung derartiger Mittel.

Bekanntlich beruht die klassische Technik zur Durchführung einer dauerhaften Haarverformung auf zwei Behandlungsschritten: Im ersten Schritt werden die Cystin-Disulfid-Brücken des Haarkeratins durch Einwirken eines Mittels, welches einen reduzierenden Wirkstoff enthält (Verformungsmittel), geöffnet. Sodann wird das Haar in die gewünschte Form gebracht. In einem zweiten Schritt werden Cystin-Disulfid-Bindungen unter Verwendung eines Fixiermittels, d.h. eines einen oxidierenden Wirkstoff enthaltenden Mittels, wieder geschlossen.

Als klassisches Dauerwellreduktionsmittel wird, wie die Pionierarbeiten in den deutschen Patentschriften 948 186 und 972 424 zeigen, die Thioglykolsäure, z.B. als Ammonium- oder Monoethanolaminsalz, verwendet. Weitere übliche reduzierende Wirkstoffe sind anorganische Sulfite, 2-Mercapto-propionsäure (Thiomilchsäure), 3-Mercapto-propionsäure, bestimmte Mercaptocarbonsäureester, Cystein und Derivate dieser Verbindungen.

Alle diese Mittel weisen jedoch eine Reihe von Nachteilen auf. Alkalisch eingestellte Zubereitungen auf Basis der Mercaptocarbonsäuren zeigen trotz ausreichender Wirkung eine Haarschädigung, die sich beispielsweise in vermehrt auftretendem Haarbruch äußert. Vielfach belasten diese Mittel auch in unerwünschter Weise die Kopfhaut.

Schließlich erfordert der unangehme Geruch der verwendeten Reduktionsmittel eine intensive Parfümierung der Produkte. Durch Verwendung von 2-Mercapto-propionsäure (Thiomilchsäure) ist man in der Lage, einige der erwähnten Probleme zu lösen. Allerdings zeichnet sich die Thiomilchsäure im Vergleich zur allgemein verwendeten Thioglykolsäure durch eine schwächere Umformung aus.

Die Mercaptocarbonsäureester, welche eine Haarverformung auch bei niedrigeren pH - Werten ermöglichen, sind bezüglich ihrer Hautverträglichkeit sowie ihres Sensibilisierungsrisikos nicht zufriedenstellend. Anstelle der Mercaptocarbonsäureester wurden auch Mercaptocarbonsäureamide wie Thioglykolsäureamid oder alkyl- bzw. hydroxyalkylsubstituierte Amide verwendet. Derartige Verbindungen sind aus den Patentschriften WO-A-91/10421 und EP-A-0 455 457 bekannt. Diese Stoffe haben, wie die Carbonsäureester, ein hohes Umformungspotential auch bei niedrigen pH-Werten , sind jedoch in Bezug auf die Sensibilisierung noch kritischer als die Ester.

In der US-A-4 220 602 ist N-Carboxymethyl-mercapto-acetamit als Dauerwellwirkstoff genannt.

Es wurde nun überraschend gefunden, daß sich die genannten Nachteile durch die Verwendung der genannten Mercaptoacetamide auf Basis der erfindungsgemäßen verzweigtkettigen Amine vermeiden lassen und daß diese über ein stärkeres Umformungspotential als Thiomilchsäure verfügen.

Gegenstand der vorliegenden Erfindung ist daher ein Mittel zur dauerhaften Verformung der Haare, welches dadurch gekennzeichnet ist, daß es als keratinreduzierenden Wirkstoff eine Verbindung der allgemeinen Formel

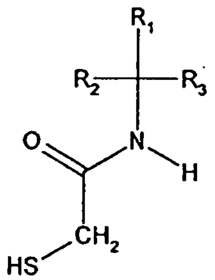

(I)

enthält, wobei $R_1$, $R_2$ und $R_3$ jeweils die Bedeutung H, Carboxy odergeradkettiger oder verzweigter Alkylrest, Monohydroxyalkylrest oder Polyhydroxyalkylrest mit 1 bis 6 Kohlenstoffatomen haben, mit der Maßgabe, daß

- nicht alle Reste $R_1$ bis $R_3$ gleichzeitig H bedeuten,
- wenn $R_1$ und $R_2$ beide H bedeuten, $R_3$ ein verzweigter Alkyl-, Monohydroxyalkyl- oder Polyhydroxyalkylrest mit 1 bis 6 Kohlenstoffatomen ist,
- wenn keiner der Reste $R_1$ bis $R_3$ die Bedeutung Carboxy oder ein verzweigter Alkyl-, Monohydroxyalkyl- oder Polyhydroxyalkylrest mit 1 bis 6 Kohlenstoffatomen hat, mindestens zwei der Reste $R_1$, $R_2$ oder $R_3$ die Bedeutung geradkettiger Alkyl-, Monohydroxyalkyl- oder Polyhydroxyalkylrest mit 1 bis 6 Kohlenstoffatomen haben und

- wenn einer der Reste Carboxy ist, die anderen beiden Reste nicht gleichzeitig Carboxy sind.

Bevorzugte Verbindungen der Formel (I) sind solche, in denen $R_1$, $R_2$ und $R_3$ jeweils die Bedeutung H, $CH_3$, $CH_2CH_3$, $CH_2CH_2CH_3$, $CH(CH_3)_2$, $C(CH_3)_3$, $CH_2CH(CH_3)CH_3$, $CH(OH)CH_3$, $CH_2OH$, $CH_2CH_2OH$, $CH_2CH(OH)CH_3$, $CH_2CH_2CH_2OH$, $CH_2CH(OH)CH_2OH$, $CH(CH_3)(CH_2OH)$, $CH(CH_2OH)_2$ oder COOH haben.

Besonders bevorzugte Verbindungen sind solche der Formeln

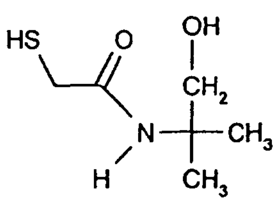

oder

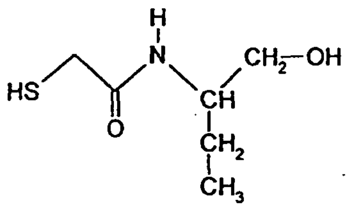

Die Herstellung der erfindungsgemäßen Mercaptoacetamide erfolgt durch Umsetzung der entsprechenden Amine mit Methylthioglykolat unter Schutzgasatmosphäre, Extraktion in geeignetem Lösungsmittel und anschliessende Kurzwegdestillation.

Die erfindungsgemäßen Mercaptoacetamide werden in dem gebrauchsfertigen Mittel zur dauerhaften Haarverformung in einer Menge von 3 bis 28 Gewichtsprozent, vorzugsweise 5 bis 21 Gewichtsprozent, eingesetzt.

Die erfindungsgemäßen Mercaptoacetamide können in einer weiteren Ausführungsform der Erfindung auch im Gemisch mit anderen, bekannten Thiolen wie Thiogtykolsäure, Thiomilchsäure, Cystein, Cysteamin und Alkyl- oder Acylcysteaminen oder Sulfiten eingesetzt werden.

Die gebrauchsfertigen Haarverformungsmittel besitzen bevorzugt einen pH-Wert von 6,5 bis 9,5 besonders bevorzugt von 6,5 bis 8,5. Als Alkalisierungsmittel bzw. als Mittel zur Einstellung des pH-Wertes kommen insbesondere Ammoniak oder Natronlauge, aber auch alle anderen wasserlöslichen, physiologisch verträglichen Salze von organischen und anorganischen Basen, wie z.B. Ammoniumhydrogencarbonat, in Betracht.

Das Verformungsmittel kann sowohl ein- als auch zweikomponentig verpackt angeboten werden. Liegt es zweikomponentig vor, dann werden die beiden Komponenten unmittelbar vor dem Gebrauch miteinander vermischt. Das Mittel kann sowohl in Form einer wäßrigen Lösung oder einer Emulsion als auch in verdickter Form auf wäßriger Basis, insbesondere als Creme, Gel, Schaum oder Paste vorliegen.

Selbstverständlich kann das Verformungsmittel alle für derartige Mittel üblichen und bekannten Zusatzstoffe, zum Beispiel Verdickungsmittel, wie beispielsweise Bentonit, Fettsäuren, Stärke, Polyacrylsäure und deren Derivate, Cellulosederivate, Alginate, Vaseline, Paraffinöle; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester; ferner Trübungsmittel, wie zum Beispiel Polyethylenglykolester; Alkohole, wie zum Beispiel Ethanol, Propanol, Isopropanol und Glycerin; Zucker wie z. B. D-Glucose; Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure und Betain, enthalten.

Die erwähnten Bestandteile werden in den für solche Zwecke üblichen Mengen verwendet, zum Beispiel die Netzmittel und Emulgatoren in Konzentrationen von insgesamt 0,2 bis 30 Gewichtsprozent, die Alkohole in einer Menge von insgesamt 0,1 bis 20 Gewichtsprozent, die Trübungsmittel, Parfümöle und Farbstoffe in einer Menge von jeweils 0,01 bis 1 Gewichtsprozent, die Puffersubstanzen in einer Menge von insgesamt 0,1 bis 10 Gewichtsprozent, Zucker, Lösungsvermittler, Stabilisatoren, sowie haarkonditionierende und haarpflegende Bestandteile in einer Menge von jeweils 0,1 bis 5 Gewichtsprozent, während die Verdickungsmittel und Lösungsvermittler in einer Menge von insgesamt 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 1 bis 30 Gewichtsprozent sowie zur Vermeidung einer Überkrausung der Haare Dithioverbindungen, beispielsweise Dithiodiglykolsäure, Dithiomilchsäure, die Dithiole der Verbindungen nach Formel (1) oder die jeweiligen Salze der Dithiole, zugesetzt werden.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte und gleichmäßige Umformung vom Haaransatz bis zur Haarspitze, ohne allergische oder sensibilisierende Reaktionen hervorzurufen.

Die vorliegende Erfindung betrifft weiterhin ein Verfahren zur dauerhaften Haarverformung, bei dem man die

Haare bevor und/oder nachdem man sie in die gewünschte Form bringt, mit einem Verformungsmittel behandelt, mit Wasser spült, sodann oxidativ behandelt, mit Wasser spült, gegebenenfalls zur Wasserwelle legt und sodann trocknet, welches dadurch gekennzeichnet ist, daß als Verformungsmittel die vorstehend beschriebenen erfindungsgemäßen Mittel verwendet werden.

Bei einer bevorzugten Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignetes Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Hydrogenperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.% in dem Nachbehandlungsmittel enthalten sein.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen den Gegenstand der Erfindung näher erläutern, ohne jedoch den Gegenstand auf diese Beispiele zu beschränken.

**Beispiele**

**Beispiel 1:**

Die Herstellung der neuen verzweigtkettig alkylsubstituierten Mercaptoacetamide erfolgt, indem man das jeweilige primäre Amin entweder nach Methode A bei einer Temperatur nicht über 30 Grad Celsius mit Methylthioglykolat umsetzt oder nach Methode B zunächst bei einer Temperatur nicht über 5 Grad Celsius mit Chloracetylchlorid und anschließend bei Raumtemperatur mit Kaliumethylxanthogenat umsetzt. Nach der Isolierung des Reaktionsprodukts erfolgt die Reinigung vorzugsweise durch Kurzwegdestillation.

Die Analysenergebnisse der Produkte der Umsetzungen sind in der nachfolgenden Tabelle 1 wiedergegeben.

**Herstellung der Mercaptoacetamide nach Methode A**

In einem 500 ml Dreihalskolben werden 2 mol des jeweiligen primären Amins vorgelegt. Unter Kühlung mit einem Wasserbad werden langsam 1 mol Methylthioglykolat derart zugetropft, daß die Temperatur 30°C nicht übersteigt. Der Ansatz wird mit Argon durchspült und solange gerührt, bis das Methylthioglykolat quantitativ umgesetzt ist (Kontrolle durch Dünnschichtchromatographie auf Merck-DC-Alufolien 5x10 cm; Kieselgel 60 F 254).

Das Gemisch wird unter Eiskühlung mit 36 %iger Salzsäure angesäuert (pH 2 - 4) und mit Ethylacetat erschöpfend extrahiert. Das Lösungsmittel wird am Umlaufverdampfer im Vakuum abdestilliert, der Rückstand durch Zugabe von Natronlauge auf pH 7,0 gebracht und nochmals mit Ethylacetat ausgeschüttelt. Die vereinigten Fraktionen werden über Natriumsulfat getrocknet und eingeengt. Der vorliegende Ruckstand wird mittels einer Kurzwegdestillationsapparatur bei höchsten 0,01 Torr destilliert zum weitestgehend reinen Produkt. Dieser Verfahrensweise kommt entscheidende Bedeutung für die Erzielung eines möglichst reinen Produktes in guter Ausbeute zu. Verunreinigungen durch nicht vollständig umgesetzte Spaltprodukte aus Thermolyse oder Hydrolyse sind wegen der sensibilisierenden Eigenschaften derselben nur durch sorgfältiges Destillieren zu vermeiden.

**Herstellung der Mercaptoacetamide nach Methode B**

In einem 1 Liter-Dreihalskolben wird ein mol des jeweiligen primären Amins in 500 ml Wasser (Aminosäuren in 500 ml 1N-NaOH) gelöst und in einem Eis-Wasserbad auf 0°C gekühlt. Die Lösung wird mit 250 ml 2N-NaOH versetzt und ein mol Chloracetylchlorid derart zugetropft, daß die Temperatur 5°C nicht übersteigt. Der Ansatz wird drei Stunden lang bei Raumtemperatur kräftig gerührt. Danach wird das Gemisch mit ein mol Kaliumethylxanthogenat versetzt und weitere zwölf Stunden bei Raumtemperatur gerührt. Das Gemisch wird mit 36%iger Salzsäure solange angesäuert, bis sich ein gelbes Öl abscheidet. Diese Öl wird abgetrennt und in einem Gemisch aus 500 ml 25%igem Ammoniak und 250 ml Ethanol gelöst. Es wird eine Stunde bei Raumtemperatur gerührt. Nachfolgend wird der Ethanol am Umlaufverdampfer im Vakuum abdestilliert, und der Rückstand mit Ethylacetat ausgeschüttelt. Die wäßrige Phase wird vorsichtig angesäuert und nochmals mit Ethylacetat extrahiert. Das Lösungsmittel wird am Umlaufverdampfer im Vakuum abdestilliert; der Rückstand wird abschließend destillativ gereinigt (siehe Methode A) oder aus Ethylacetat umkristallisiert.

**Tabelle 1**

| **Mercaptoacetamide** | | | | | | | | |
|---|---|---|---|---|---|---|---|---|
| **Aminkomponente** | **Ausbeute in %** | **Elementaranalyse ber/gef** | **HPLC (FP)** | **Siedepunkt** | **WSN pH = 7** | **WSN pH = 8** | **WSN pH = 9** | **Synthese methode** |
| 1) N-(2'-Hydroxy-tert-butyl)-2-mercaptoacetamid<br>2-Amino-2-methylpropanol | 74 | C: 44,15, H: 8,03, N: 8,58, S: 19,64, C: 43,75, H: 7,99, N: 8,62, S: 19,61 | 90,778 | 117° C/ 0,01 Torr | 81 | 98 | 102 | A |
| 2) N-2'-(1-Hydroxybutyl)-2-mercaptoacetamid<br>2-Amino-1-butanol | 59 | C: 44,15, H: 8,03, N: 8,58, S: 19,64, C: 44,22, H: 8,00, N: 8,36, S: 19,40 | 97,375 | 121°C/ 0,01 Torr | 78 | 83 | 97 | A |
| 3) N-(2'-Butyl)-2-mercaptoacetamid<br>Isobutylamin | 85 | C: 48,95, H: 8,90 N: 9,51, S: 21,77, C: 48,61, H: 8,59, N: 9,26, S: 21,60 | 98,25 | 85° C/ 0,01 Torr | 79 | 88 | 96 | A |
| 4) N-[1,1-bis (Hydroxymethyl)ethyl)]-2-mercaptoacetamid<br>2-Amino-2-methyl-1,3-propandiol | 35 | C: 40,21, H: 7,31, N: 7,81, S: 17,89, C: 39,71, H: 6,73, N: 7,77, S: 18,13 | 98,12 | Fp: 89° C | 80 | 94 | 96 | A |
| 5) N-Isopropyl-2-mercaptoacetamid<br>Isopropylamin | 70 | C: 45,08, H: 8,32, N: 10,51, S: 24,07, C: 44,96, H: 7,99, N: 10,15, S: 23,08 | 98,111 | 71° C/- 0.075 | 78 | 74 | 98 | A |
| 6) N-tert-Butyl-2-mercaptoacetamid<br>tert-Butylamin | 26 | C: 48,95, H: 8,90, N: 9,51, S: 21,77, C: 48,58, H: 8,61, N: 9,725, S: 22,06 | 97,13 | 62° C | 64 | 74 | 96 | B |

(fortgesetzt)

| Mercaptoacetamide | | | | | | | | |
|---|---|---|---|---|---|---|---|---|
| **Aminkomponente** | **Ausbeute in %** | **Elementaranalyse ber/gef** | **HPLC (FP)** | **Siedepunkt** | **WSN pH = 7** | **WSN pH = 8** | **WSN pH = 9** | **Synthese methode** |
| 7) N-(2-Mercaptoacetyl)-Leucin<br><br>Leucin | 77 | C: 46,81, H: 7,37, N: 6,82, S: 15,62, C: 46,84, H: 7,22, N: 7,01, S: 15,70 | 96,22 | 138°C | 53 | 68 | 69 | B |
| 8) N-Isobutyl-2'-mercaptoacetamid<br><br>Isobutylamin | 61 | C: 48,95, H: 8,90, N: 9,51, S: 21,77, C: 48,77, H: 8,69, N: 9,50, S: 21,69 | 97,95 | 97° C/0.1 | 79 | 86 | 102 | A |
| Thiomilchsäure als Vergleich | | | | | 57 | 50 | 70 | |

**Beispiel 2: Herstellung von N-(2'-Butyl)-2-mercaptoacetamid**

In einem 500-ml-Dreihalskolben werden 146,26 g (2 mol) Isobutylamin vorgelegt. Es werden langsam 106,24 g Methylthioglykolat derart zugetropft, daß die Temperatur 30° C nicht übersteigt. Der Ansatz wird mit Argon durchspült und 2 Tage bei Raumtemperatur gerührt.

Das Gemisch wird unter Eiskühlung mit 36prozentiger Salzsäure angesäuert (pH 2 - 4) und mit Ethylacetat erschöpfend extrahiert. Das Lösungsmittel wird am Umlaufverdampfer im Vakuum abdestilliert, der Rückstand wird durch Zugabe von Natronlauge auf pH 7,0 gebracht und nochmals mit Ethylacetat ausgeschüttelt. Die vereinigten Fraktionen werden über Natriumsulfat getrocknet und eingeengt. Der vorliegende Rückstand wird mittels einer Kurzwegdestillationsapparatur bei höchstens 0,01 Torr zum reinen Produkt destilliert. Die Ausbeute beträgt 125 g (85 %).

Analytik:

a) $^{1}$H-NMR ($CDCl_3$):

| | | |
|---|---|---|
| δ (ppm) = | 6,49 | (bauchig, -NH) |
| | 3,905 | (m, 4H, + NH-CH) |
| | 3,23 | (d, 2H, HS-$CH_2$-CO) |
| | 1,89 | (bauchig, 1H, HS) |
| | 1,505 | (m, 2H, NH-CH-$CH_2$) |
| | 1,16 | (m, 3H, NH-CH-$CH_3$) |
| | 0,92 | (t, 3H, $CH_2$-$CH_3$) |

b) $^{13}$C-NMR ($CDCl_3$):

| | | |
|---|---|---|
| δ (ppm) = | 168,43 | (-C = O) |
| | 47,08 | (NH-CH) |
| | 29,51 | (NH-CH-$CH_2$-$CH_3$) |
| | 28,43 | (HS-$CH_2$) |
| | 20,32 | (CH-$CH_3$) |
| | 10,41 | ($CH_2$-$CH_3$) |

c) MS (70 e V, EI, RT)

m/z (%) = ($M^+$) = 147 (60,86)
132(2,26), 114(15,61), 104(18,59), 100(15,26), 92 (15,41), 91 (10,39), 72 (14,51), 57 (100)

d) Thioltitration: 96,35 %
e) Elementaranalyse: $C_6H_{13}NOS$ (MG: 147,24)

| | | | | |
|---|---|---|---|---|
| Ber.: | C 48,95, | H 8,90, | N 9,51, | S 21,77 |
| Gef.: | C 48,61, | H 8,59, | N 9,26, | S 21,60 |

f) IR (NaCl-Platten):

| | |
|---|---|
| 3294s | (NH) |
| 3075-2878s | ($CH_2$) |
| 2555w | (SH) |
| 1646s | (N-monosubstituiertes Amid) |
| 1559s | (N-monosubstituiertes Amid) |

g) HPLC: Die HPLC ergab ein Ergebnis von 98,25 Flächenprozent für die Verbindung.

(Säule: C 18 5U, 250 mm x 4,6 mm; Fließmittel Acetonitril: Puffer [4 g $KH_2PO4$ + 0,8 g Octansulfonsäure-Na-Salz + 2 ml $H_3PO_4$] = 25 : 75; Flußrate 0,5 ml/min; Wellenlänge 200 nm;)

h) pKs: 7,357 ($H_2O$)

i) UV-max: 201 nm (Acetonitril: Puffer = 25 : 75)

j) Siedepunkt: 85 °C/0,01 Torr

**Beispiel 3: Herstellung von N-(2'-Hydroxy-tert-butyl)-2-mercaptoacetamid**

In einem 500-ml-Dreihalskolben werden 178,19 g (2 mol) 2-Amino-2-methyl-propanol vorgelegt.

Es werden langsam 106,24 Methylthioglykolat derart zugetropft, daß die Temperatur 30°C nicht übersteigt. Der Ansatz wird mit Argon durchspült und 2 Tage bei Raumtemperatur gerührt.

Das Gemisch wird unter Eiskühlung mit 36prozentiger Salzsäure angesäuert (pH 2 - 4) und mit Ethylacetat erschöpfend extrahiert. Das Lösungsmittel wird am Umlaufverdampfer im Vakuum abdestilliert, der Rückstand wird durch Zugabe von Natronlauge auf pH 7,0 gebracht und nochmals mit Ethylacetat ausgeschüttelt. Die vereinigten Fraktionen werden über Natriumsulfat getrocknet und eingeengt. Der vorliegende Rückstand wird mittels einer Kurzwegdestillationsapparatur bei höchstens 0,01 Torr zum reinen Produkt destilliert. Die Ausbeute beträgt 121 g (74 %).

Analytik:

a) $^1H$-NMR ($CDCl_3$):

| | | | |
|---|---|---|---|
| δ (ppm) = | 6,81 | bs, 1H | NH |
| | 4,55 | bs, 1H | OH |
| | 3,6 | s, 2H | $CH_2$-OH |
| | 3,21 | s, 2H | 2 - H |
| | 1,95 | bs, 1H | HS |
| | 1,32 | s, 6H | $CH_3$ |

b) $^{13}C$-NMR ($CDCl_3$):

| | | |
|---|---|---|
| δ (ppm) = | 170,07 | (C-1) |
| | 70,07 | ($CH_2$-OH) |
| | 56,17 | (NH-C) |
| | 28,77 | (C-2) |
| | 24,31 | ($CH_3$) |

c) MS (70 e V, EI, 50 °C)

m/z (%) = ($M^+$) = 163 (4,$M^+$)
132 (100), 105 (100), 92 (13), 73 (28), 58 (92), 55 (17)

d) Thioltitration: 96,81 %

e) Elementaranalyse: $C_6H_{13}NO_2S$ (MG: 163,23 g/mol)

| | | | | |
|---|---|---|---|---|
| Ber.: | C 44,15; | H 8,03; | N 8,58; | S 19,64 |
| Gef.: | C 43,75; | H 7,99: | N 8,62; | S 19,61 |

f) IR (NaCl-Platten):

| | |
|---|---|
| 3304s | (OH) |
| 3084-2933m | ($CH_2$) |
| 2556w | (SH) |

(fortgesetzt)

| | |
|---|---|
| 1654s | (N-monosubstituiertes Amid) |
| 1559s | (N-monosubstituiertes Amid) |

g) HPLC: Die HPLC ergab ein Ergebnis von 90,778 Flächenprozent für die Verbindung.
(Säule: C 18 5U, 250 mm x 4,6 mm; Fließmittel Acetonitril: Puffer [4 g $KH_2PO4$ + 0,8 g Octansulfonsäure-Na-Salz + 2 ml $H_3PO_4$] = 25 : 75; Flußrate 0,5 ml/min; Wellenlänge 200 nm;)
h) pKs: 7,96 ($H_2O$)
i) UV-max: < 207,8 nm (Acetonitril: Puffer = 25 : 75)
j) Siedepunkt: 117°C/0,01 Torr

**Beispiel 4: Vergleich der Wellwirksamkeit**

Die Wellwirksamkeit der 1-Mercaptoacetamide wurde unter Verwendung von Glycerinmonothioglykolat als Vergleichssubstanz mit Hilfe von Welllösungen bei pH = 7, 8 und 9 bestimmt. Hierzu wurden 16,5 Zentimeter lange, vorgebleichte und damit geschädigte Zählhaarsträhnen (bestehend aus ca. 100 Haaren) aus mitteleuropäischem Haar naß auf genormte Spiralwickler (Innendurchmesser: 3 Millimeter) aufgewickelt und nach dem Konditionieren im Klimaraum (Temperatur: 20°C; Luftfeuchte: 65%) mit einer 87 mmol/100g enthaltenden, auf den jeweiligen pH-Wert eingestellten Lösung der Reduktionsmittel behandelt. Die Auftragemenge an Wellflüssigkeit wurde über das Verhältnis 1 : 1,2 errechnet (1 g Haar: 1,2 ml Wellflüssigkeit). Als Einwirkzeit wurden 20 Minuten gewählt; die Einwirktemperatur betrug 50 Grad Celsius. Anschließend wurden die Haare mit einer peroxidhaltigen Fixierung fixiert, getrocknet und nach dem Abwickeln vier Stunden in Wasser (Wasserbadtemperatur: 40°C) ausgehängt.
Die Wellstabilität errechnet sich gemäß folgender Formel:

$$\text{Wellstabilität in \%} = \frac{l_o - l_t}{l_o - l_1} \times 100$$

$l_o$ = Gesamtlänge der nicht umgeformten, gestreckten Strähne (16,5 Zentimeter)
$l_t$ = Länge der abgewickelten, ausgehängten Strähne nach 240 Minuten
$l_1$ = Länge der umgeformten, aufgewickelten Strähne
bei einem Wickelinnendurchmesser von 3 mm beträgt diese: $l_1$ = 35 Millimeter

Als Standard wurden Strähnchen mit einer entsprechend auf pH 9 eingestellten Glycerinmonothioglykolat-Lösung behandelt. Die vorstehend in Tabelle 1 angegebenen normierten Wellstabilitäten beziehen sich auf diese Standardlösung (pH = 9), deren Wellstabilität auf 100 Prozent gesetzt wurde.
Tabelle 1 zeigt, daß die Wellwirksamkeiten der erfindungsgemäßen Mercaptoacetamide bei pH-Werten von 7, 8 und 9 höher sind als bei Thiomilchsäure

**Beispiel 5: Dauerverformungsmittel für gefärbtes Haar**

| | |
|---|---|
| 12,0 g | N-(2'-Hydroxy-tert-butyl)-2-mercaptoacetamid |
| 0,4 g | Ammoniak (25%-ige wäßrige Lösung) zur pH-Einstellung |
| 2,0 g | Ammoniumhydrogencarbonat |
| 2,0 g | Isopropanol |
| 1,0 g | Isooctylphenol, oxethyliert mit 10 Mol Ethylenoxid |
| 1,0 g | Poly(dimethyldiallylammoniumchlorid) |
| 0,3 g | Parfümöl |
| 0,1 g | Vinylpyrrolidon/Styrol-Mischpolymerisat (Antara® 430 der GAF Corp.; New York/USA) |

(fortgesetzt)

| | |
|---|---|
| 81,2 g | Wasser |
| 100,0 g | |

Der pH-Wert dieses Mittels liegt bei 7,0 bis 7,5.

Durch Farbbehandlungen vorgeschädigtes Haar wird mit einem Shampoo gewaschen, frottiert und auf Wickler mit einem Durchmesser von 8 Millimetern gewickelt. Anschließend wird das vorstehend beschriebene Haarverformungsmittel gleichmäßig auf dem gewickelten Haar verteilt. Sodann wird das Haar mit einer Plastikhaube abgedeckt und 10 Minuten lang unter einer Trockenhaube bei einer Temperatur von 45 Grad Celsius erwärmt. Anschließend wird die Abdeckung entfernt, das Haar mit Wasser gespült und mit 100 Gramm einer 3-prozentigen wäßrigen Hydrogenperoxidlösung oxidativ nachbehandelt. Nach Entfernung der Wickler werden die Haare erneut mit Wasser gespült, zur Wasserwelle gelegt und sodann getrocknet.

Als Ergebnis dieser Behandlung wird eine gleichmäßige, elastische und dauerhafte Verformung der Haare erhalten.

**Beispiel 6: Dauerwellverformungsmittel für normales Haar**

| | |
|---|---|
| 17,5 | N-(2'-Butyl)-2-mercaptoacetamid |
| 8,9 g | Ammoniak (25%-ige wäßrige Lösung) |
| 5,0 g | Ammoniumhydrogencarbonat |
| 4,0 g | Harnstoff |
| 2,4 g | Monoethanolamin |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat (Antara® 430 der GAF Corp.; New York/USA) |
| 59,6 g | Wasser |
| 100,0 g | |

Der pH-Wert dieses Mittels beträgt 8,4.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

**Beispiel 7: Dauerwellverformungsmittel für normales Haar**

| | |
|---|---|
| 17,5 g | N-2'-(1-Hydroxybutyl)-2-mercaptoacetamid |
| 8,9 g | Ammoniak (25%-ige wäßrige Lösung) zur pH-Einstellung |
| 5,0 g | Ammoniumhydrogencarbonat |
| 2,0 g | D-Glucose |
| 2,4 g | Ammoniak |
| 1,5 g | Isooctylphenol, oxethyliert mit 10 Mol Ethylenoxid |
| 0,5 g | Poly(dimethyldiallylammoniumchlorid) |
| 0,5 g | Parfümöl |
| 0,1 g | Vinylpyrrolidon/Styrol-Mischpolymerisat |

(fortgesetzt)

| | |
|---|---|
| | (Antara® 430 der GAF Corp.; New York/USA) |
| 61,6 g | Wasser |
| 100,0 g | |

Der pH dieses Mittels liegt bei 8,0 bis 8,5.

Normales, nicht vorgeschädigtes Haar wird gewaschen, mit einem Handtuch frottiert und auf Wickler mit einem Durchmesser von 6 Millimetern gewickelt. Anschließend wird das Haar mit dem vorstehend beschriebenen Haarverformungsmittel gleichmäßig durchfeuchtet. Nach einer Einwirkungszeit von 15 - 25 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Das so behandelte Haar besitzt eine gleichmäßige und lebhafte Krause.

## Patentansprüche

1. Mittel zur dauerhaften Verformung von Haaren, **dadurch gekennzeichnet, daß** es als keratinreduzierenden Wirkstoff eine Verbindung der allgemeinen Formel

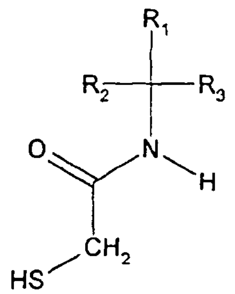

(I)

enthält, wobei $R_1$, $R_2$ und $R_3$ jeweils die Bedeutung H, Carboxy odergeradkettiger oder verzweigter Alkylrest, Monohydroxyalkylrest oder Polyhydroxyalkylrest mit 1 bis 6 Kohlenstoffatomen haben, mit der Maßgabe, daß

- nicht alle Reste $R_1$ bis $R_3$ gleichzeitig H bedeuten,
- wenn $R_1$ und $R_2$ beide H bedeuten, $R_3$ Carboxy oder ein verzweigter Alkyl-, Monohydroxyalkyl- oder Polyhydroxyalkylrest mit 1 bis 6 Kohlenstoffatomen ist,
- wenn keiner der Reste $R_1$ bis $R_3$ die Bedeutung Carboxy oder ein verzweigter Alkyl-, Monohydroxyalkyl- oder Polyhydroxyalkylrest mit 1 bis 6 Kohlenstoffatomen hat, mindestens zwei der Reste $R_1$, $R_2$ oder $R_3$ die Bedeutung geradkettiger Alkyl-, Monohydroxyalkyl- oder Polyhydroxyalkylrest mit 1 bis 6 Kohlenstoffatomen haben und
- wenn einer der Reste Carboxy ist, die anderen beiden Reste nicht gleichzeitig Carboxy sind.

2. Mittel nach Anspruch 1, **dadurch gekennzeichnet, daß** in der Formel (I) $R_1$, $R_2$ und $R_3$ jeweils die Bedeutung H , $CH_3$ , $CH_2CH_3$, $CH_2CH_2CH_3$, $CH(CH_3)_2$, $C(CH_3)_3$, $CH_2CH(CH_3)CH_3$, $CH(OH)CH_3$, $CH_2OH$, $CH_2CH_2OH$, $CH_2CH(OH)CH_3$, $CH_2CH_2CH_2OH$, $CH_2CH(OH)CH_2OH$, $CH(CH_3)(CH_2OH)$ $CH(CH_2OH)_2$ oder COOH haben.

3. Mittel nach einem der Ansprüche 1 oder 2, **dadurch gekennzeichnet, daß** die Verbindung der Formel (I) in dem gebrauchsfertigen Mittel in einer Menge von 3 bis 28 Gewichtsprozent enthalten ist.

4. Mittel nach einem der Ansprüche 1 bis 3, **dadurch gekennzeichnet, daß** der pH-Wert des gebrauchsfertigen Mittels 6,5 bis 9,5 beträgt.

5. Mittel nach einem der Ansprüche 1 bis 4, **dadurch gekennzeichnet, daß** es vor der Anwendung durch Vermischen von zwei oder drei Komponenten erhalten wird.

6. Verfahren zur dauerhaften Verformung von Haaren, bei dem man das Haar bevor und/oder nachdem man es in

der gewünschten Form festhält, mit einem Verformungsmittel behandelt, mit Wasser spült, oxidativ nachbehandelt, erneut mit Wasser spült, gegebenenfalls zur Wasserwelle legt und sodann trocknet, **dadurch gekennzeichnet, daß** man als Verformungsmittel ein Mittel nach einem der Ansprüche 1 bis 5 verwendet.

**7.** Verfahren nach Anspruch 6, **dadurch gekennzeichnet, daß** man das Verformungsmittel 5 bis 30 Minuten lang einwirken läßt.

**8.** Verfahren nach Anspruch 6, **dadurch gekennzeichnet, daß** man das Verformungsmittel unter Anwendung von Wärme 5 bis 20 Minuten lang einwirken läßt.

**9.** Verfahren nach einem der Ansprüche 6 bis 8, **dadurch gekennzeichnet, daß** man das Verformungsmittel in einer Menge von 60 bis 120 Gramm anwendet.

**10.** Verfahren zur Herstellung von Mercaptoacetamiden der Formel (1), **daduch gekennzeichnet**, **daß** man das jeweilige primäre Amin bei einer Temperatur nicht über 30 Grad Celsius mit Methylthioglykolat umsetzt.

**Claims**

**1.** Agent for permanently shaping hair, **characterized in that** it comprises, as keratin-reducing active ingredient, a compound of the general formula

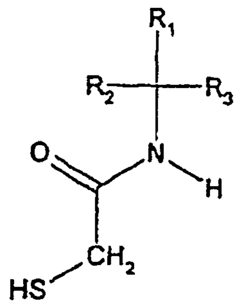

(I)

where $R_1$, $R_2$ and $R_3$ in each case have the meaning H, carboxy or straight-chain or branched alkyl radical, monohydroxyalkyl radical or polyhydroxyalkyl radical having 1 to 6 carbon atoms, with the proviso that

- not all radicals $R_1$ to $R_3$ are H at the same time,
- if $R_1$ and $R_2$ are both H, $R_3$ is carboxy or a branched alkyl, monohydroxyalkyl or polyhydroxyalkyl radical having 1 to 6 carbon atoms,
- if none of the radicals $R_1$ to $R_3$ have the meaning carboxy or a branched alkyl, monohydroxyalkyl or polyhydroxyalkyl radical having 1 to 6 carbon atoms, at least two of the radicals $R_1$, $R_2$ or $R_3$ have the meaning straight-chain alkyl, monohydroxyalkyl or polyhydroxyalkyl radical having 1 to 6 carbon atoms and
- if one of the radicals is carboxy, the other two radicals are not carboxy at the same time.

**2.** Agent according to Claim 1, **characterized in that**, in the formula (I), $R_1$, $R_2$ and $R_3$ in each case have the meaning H, $CH_3$, $CH_2CH_3$, $CH_2CH_2CH_3$, $CH(CH_3)_2$, $C(CH_3)_3$, $CH_2CH(CH_3)CH_3$, $CH(OH)CH_3$, $CH_2OH$, $CH_2CH_2OH$, $CH_2CH(OH)CH_3$, $CH_2CH_2CH_2OH$, $CH_2CH(OH)CH_2OH$, $CH(CH_3)(CH_2OH)$, $CH(CH_2OH)_2$ or COOH.

**3.** Agent according to one of Claims 1 or 2, **characterized in that** the compound of the formula (I) is present in the ready-to-use agent in an amount of from 3 to 28% by weight.

**4.** Agent according to one of Claims 1 to 3, **characterized in that** the pH of the ready-to-use agent is 6.5 to 9.5.

**5.** Agent according to one of Claims 1 to 4, **characterized in that** it is obtained prior to application by mixing two or three components.

**6.** Method for permanently shaping hair, in which the hair, before and/or after it is held in the desired shape, is treated with a shaping agent, rinsed with water, oxidatively after-treated, rinsed again with water, optionally set and then dried, **characterized in that** the shaping agent used is an agent according to one of Claims 1 to 5.

**7.** Method according to Claim 6, **characterized in that** the shaping agent is left to act for 5 to 30 minutes.

**8.** Method according to Claim 6, **characterized in that** the shaping agent is left to act for 5 to 20 minutes with the application of heat.

**9.** Method according to one of Claims 6 to 8, **characterized in that** the shaping agent is applied in an amount of from 60 to 120 grams.

**10.** Method of preparing mercaptoacetamides of the formula (I), **characterized in that** the respective primary amine is reacted with methyl thioglycolate at a temperature not exceeding 30 degrees Celsius.

## Revendications

**1.** Composition pour la mise en forme permanente des cheveux, **caractérisée en ce qu'**elle contient en tant que substance active réduisant la kératine un composé de formule générale

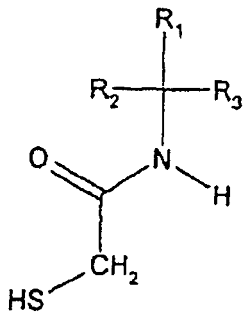

(I)

dans laquelle $R_1$, $R_2$ et $R_3$ représentent chacun H, un groupe carboxy ou un radical alkyle, monohydroxyalkyle ou polyhydroxyalkyle à chaîne droite ou ramifiée, ayant de 1 à 6 atomes de carbone, étant entendu que

- les radicaux $R_1$ à $R_3$ ne représente pas tous simultanément H,
- si $R_1$ et $R_2$ représente l'un et l'autre H, $R_3$ est un groupe carboxy ou un radical alkyle, monohydroxyalkyle ou polyhydroxyalkyle ramifié, ayant de 1 à 6 atomes de carbone,
- si aucun des radicaux $R_1$ à $R_3$ ne représente un groupe carboxy ou un radical alkyle, monohydroxyalkyle ou polyhydroxyalkyle ramifié, ayant de 1 à 6 atomes de carbone, au moins deux des radicaux $R_1$, $R_2$ et $R_3$ représentent un radical alkyle, monohydroxyalkyle ou polyhydroxyalkyle à chaîne droite ayant de 1 à 6 atomes de carbone et
- si l'un des radicaux est un groupe carboxy, les deux autres radicaux ne sont pas simultanément des groupes carboxy.

**2.** Composition selon la revendication 1, **caractérisée en ce que** dans la formule (I) $R_1$, $R_2$ et $R_3$ représentent chacun H, $CH_3$ , $CH_2CH_3$, $CH_2CH_2CH_3$, $CH(CH_3)_2$, $C(CH_3)_3$, $CH_2CH(CH_3)CH_3$, $CH(OH)CH_3$, $CH_2OH$, $CH_2CH_2OH$, $CH_2CH(OH)CH_3$, $CH_2CH_2CH_2OH$, $CH_2CH(OH)CH_2O$, $CH(CH_3)(CH_2OH)$, $CH(CH_2OH)_2$ ou COOH.

**3.** Composition selon la revendication 1 ou 2, **caractérisée en ce que** le composé de formule (I) est contenu dans la composition prête à l'emploi en une quantité de 3 à 28 % en poids.

**4.** Composition selon l'une quelconque des revendications 1 à 3, **caractérisée en ce que** le pH de la composition prête à l'emploi est de 6,5 à 9,5.

**5.** Composition selon l'une quelconque des revendications 1 à 4, **caractérisée en ce qu'**elle est obtenue avant l'emploi,

par mélange de deux ou trois composants.

6. Procédé pour la mise en forme permanente des cheveux, dans lequel les cheveux, avant et/ou après été maintenus en la forme désirée, sont traités par une composition de mise en forme, rincés à l'eau, soumis à un post-traitement par oxydation, rincés de nouveau à l'eau, éventuellement mis en plis et ensuite séchés,
**caractérisé en ce qu'**on utilise comme composition de mise en forme une composition selon l'une quelconque des revendications 1 à 5.

7. Procédé selon la revendication 6, **caractérisé en ce qu'**on laisse agir la composition de mise en forme pendant 5 à 30 minutes.

8. Procédé selon la revendication 6, **caractérisé en ce qu'**on laisse agir la composition de mise en forme, avec application de chaleur, pendant 5 à 20 minutes.

9. Procédé selon l'une quelconque des revendications 6 à 8, **caractérisé en ce qu'**on utilise la composition de mise en forme en une quantité de 60 à 120 grammes.

10. Procédé pour la préparation de mercaptoacétamides de formule (I), **caractérisé en ce qu'**on fait réagir l'amine primaire respective avec du thioglycolate de méthyle, à une température n'excédant pas 30 °C.